# EUROPEAN PATENT SPECIFICATION

(11) **EP 4 370 033 B1**
(45) Date of publication and mention of the grant of the patent: **10.09.2025**
(21) Application number: 22744524.4
(22) Date of filing: 05.07.2022
(51) Int. Cl.: A61B 7/02, A61L 29/04, A61L 29/08, A61L 31/04, A61L 31/10, B05D 1/00, C09D 165/04, B05D 1/20, B05D 7/02, B05D 7/00, C08L 65/04

(54) **PROTECTIVE COATINGS FOR PRINTED SURFACES**
SCHUTZBESCHICHTUNGEN FÜR GEDRUCKTE OBERFLÄCHEN
REVÊTEMENTS PROTECTEURS POUR SURFACES IMPRIMÉES

(30) Priority: 15.07.2021 US 202163221990 P
(43) Date of publication of application: 22.05.2024
(73) Proprietor: Solventum Intellectual Properties Company, Maplewood, MN 55144 (US)
(72) Inventor: ROGERS, Daniel J., Saint Paul, Minnesota 55133-3427 (US); SHERMAN, Audrey A., Saint Paul, Minnesota 55133-3427 (US)
(74) Representative: Vossius & Partner Patentanwälte Rechtsanwälte mbB
(86) International application number: PCT/IB2022/056223
(87) International publication number: WO 2023/285920

(56) References cited:
- EP-A1- 2 839 850
- JP-A- H07 275 262
- CHIELLINI FEDERICA ET AL: "Perspectives on alternatives to phthalate plasticized poly(vinyl chloride) in medical devices applications", PROGRESS IN POLYMER SCIENCE, PERGAMON PRESS, OXFORD, GB, vol. 38, no. 7, 19 March 2013 (2013-03-19), pages 1067 - 1088, XP028566290, ISSN: 0079-6700, DOI: 10.1016/J.PROGPOLYMSCI.2013.03.001
- M. MESSORI ET AL: "Prevention of plasticizer leaching from PVC medical devices by using organic?inorganic hybrid coatings", POLYMER, vol. 45, no. 3, 1 February 2004 (2004-02-01), AMSTERDAM, NL, pages 805 - 813, XP055560050, ISSN: 0032-3861, DOI: 10.1016/j.polymer.2003.12.006

## Description

### Summary

Disclosed herein are medical articles that have a printed surface and a transparent vapor-deposited coating of a barrier polymer layer covering and protecting the printed surface. Also disclosed are methods of preparing the medical articles.

In some embodiments, the medical article comprises a tube with an interior surface and an exterior surface where at least a portion of the exterior surface of the tube is a printed surface, and a transparent vapor-deposited coating of a barrier polymer covering at least a portion of at least the exterior surface of the tube. The tube comprises a polymer composition containing one or more extractable components. The transparent vapor-deposited coating comprises a poly(p-xylylene) polymer. The printed surface is visible through the barrier polymer layer. The vapor-deposited polymer coating barrier reduces the extraction of extractable component(s) from the tube as well as protecting the printed surface.

Also disclosed are methods of preparing medical articles. In some embodiments, the method comprises providing a tube with an interior surface and an exterior surface, comprising a polymer composition containing one or more extractable components, printing on at least a portion of the exterior surface of the tube, and vapor coating a transparent = barrier polymer comprising a poly(p-xylylene) polymer onto at least a portion of at least the exterior surface of the tube.

### Brief Description of the Drawings

The present application may be more completely understood in consideration of the following detailed description of various embodiments of the disclosure in connection with the accompanying drawings.
Figure 1 is a top view of a stethoscope.
Figure 2 is a cross-sectional view of a tubing article of this disclosure.
Figure 3 is a top view of a picture of an article of this disclosure.
Figure 4 is a top view of a picture of an article of this disclosure.

In the following description of the illustrated embodiments, reference is made to the accompanying drawings, in which is shown by way of illustration, various embodiments in which the disclosure may be practiced. It is to be understood that the embodiments may be utilized and structural changes may be made without departing from the scope of the present disclosure. The figures are not necessarily to scale. Like numbers used in the figures refer to like components. However, it will be understood that the use of a number to refer to a component in a given figure is not intended to limit the component in another figure labeled with the same number.

### Detailed Description

The scope of this invention is defined by the claims. A wide range of devices and articles are prepared from polymeric materials. Among these devices are medical devices and articles. Many of these articles and devices desirably have flexibility. Among the flexible materials used are polymers that contain extractable materials such as plasticizers, fillers, tackifiers, heat stabilizers, and the like. These extractable materials are often present in large quantities. The extractable materials can be extracted from the polymer by exposure to solvents, by heat, by contact with fluids such as aqueous or hydroalcoholic disinfectants, by high humidity (e.g. >90% relative humidity), by contact with skin, or even over time. This extraction is undesirable for many reasons and can cause difficulties in the use of the medical articles.

In M. Messori et al., Polymer, Volume 45, Issue 3, 2004, pages 805-813 the prevention of plasticizer leaching from PVC medical devices by using organic-inorganic hybrid coatings is described.

Additionally, it is often desirable that the polymeric articles and devices have printed surfaces. The printing can be for aesthetic purposes, informational purposes, or a combination thereof. Printing on flexible polymeric materials can have difficulties since the printing tends to be relatively rigid compared to the flexible polymeric materials. Thus, bending and flexing in the polymeric material can cause the printing to crack or flake off. Also, since the polymeric material contains extractable materials, the printing can be removed from the surface along with loss of extractable materials. For example, if the printed surface is wiped to clean it, the wiping motion can remove extractable material as well as the printing.

One particularly useful polymeric material is polyvinyl chloride (PVC) often referred to as "vinyl". PVC by itself is a rigid material, and thus plasticizers are added to the material to make it softer and more flexible. Among the commonly used plasticizers are phthalates. In recent years, the use of phthalate plasticizers in PVC for medical uses has fallen into disfavor, and phthalate-free PVC materials are being used more and more in medical applications. However, as is well known in the chemical arts, making a change in composition often causes changes in the properties of the materials.

PVC materials are frequently used in tubing materials, such as the sound transmitting tubes of stethoscopes. These tubes have a variety of required property features. Among these properties are flexibility, resistance to degradation from exposure to heat and chemicals, durability, an aesthetically pleasant look, and a pleasant feel. The introduction of phthalate-free PVC materials has been observed to adversely affect some or all of these properties. As the stethoscopes are used, washing, and exposure to the body heat of the user tends to cause the PVC material to become more rigid as plasticizer leaches out or is washed away.

In this disclosure, it has been found that coating of polymeric tubes that contain extractable components and have a printed surface with a vapor-deposited coating of a poly(p-xylylene) polymer not only reduces the extractability of the extractable components, but also provides a variety of additional desirable features. Among these features are optical transparency and tactile features such as the texture, and coefficient of friction. Because the coating protects the tubing, the tubing retains its desirable properties and printed surface over the lifetime of the article.

Disclosed herein are tubes useful in tubing articles, where the tubes comprise a polymer composition containing one or more extractable components with a printed coating on at least a portion of one surface of the tube, where the tube article has a vapor-deposited coating of a barrier polymer derived from at least one ethylenically unsaturated monomer on at least the exterior surface of the tube. The barrier polymer comprises a poly(p-xylylene) polymer. In some embodiments, the vapor-deposited coating is present on both the interior and exterior surface of the tube. The barrier polymer is derived from at least one ethylenically unsaturated monomer and may be optionally crosslinked by incorporation of at least one dimeric ethylenically unsaturated monomer. The vapor-deposited barrier polymer coating reduces the extraction of extractable component(s) from the tube and also protects the printed layer of the tube article. Additionally, the protective coating provides a cleanable surface. By this it is meant that if the surface becomes soiled or marked, the soiling or marking can be removed without removing or adversely affecting the printed coating. In this way, the protective coating not only protects the printed coating and reduces the extraction of extractable components from the tube, but also provides a coating surface that is cleanable. Also disclosed are methods of preparing tubing articles, such as medical articles. Among the tubing articles are stethoscopes.

The term "tube" and "tubing" as used herein refers to a three-dimensional tubular article that is cylindrically symmetric. Tubes and tubing are defined by an inner diameter, an outer diameter, (the thickness of the tubing is the difference between the outer diameter and the inner diameter) and a length. While the thickness of the tubing can vary slightly through the length of the tube as the result of the method of preparation, etc., no intentional asymmetries are present in the tubing. Typically, the length is substantially greater than the diameter of the tube.

The term "(meth)acrylate" refers to monomeric acrylic or methacrylic esters of alcohols. Acrylate and methacrylate monomers or oligomers are referred to collectively herein as "(meth)acrylates". Materials referred to as "(meth)acrylate functional" are materials that contain one or more (meth)acrylate groups.

The terms "polysiloxane" and "siloxane-based" as used herein refer to polymers or units of polymers that contain siloxane units. The terms silicone or siloxane are used interchangeably and refer to units with dialkyl or diaryl siloxane (-SiR₂O-) repeating units.

The terms "room temperature" and "ambient temperature" are used interchangeably to mean temperatures in the range of 20°C to 25°C.

Unless otherwise indicated, the terms "optically transparent", and "visible light transmissive" are used interchangeably, and refer to a layer, article, or film that has a high light transmittance over at least a portion of the visible light spectrum (about 400 to about 700 nm). Typically, optically transparent articles have a visible light transmittance of at least 90% and a haze of less than 10%.

The terms "polymer" and "macromolecule" are used herein consistent with their common usage in chemistry. Polymers and macromolecules are composed of many repeated subunits. As used herein, the term "macromolecule" is used to describe a group attached to a monomer that has multiple repeating units. The term "polymer" is used to describe the resultant material formed from a polymerization reaction.

The term "alkyl" refers to a monovalent group that is a radical of an alkane, which is a saturated hydrocarbon. The alkyl can be linear, branched, cyclic, or combinations thereof and typically has 1 to 20 carbon atoms. In some embodiments, the alkyl group contains 1 to 18, 1 to 12, 1 to 10, 1 to 8, 1 to 6, or 1 to 4 carbon atoms. Examples of alkyl groups include, but are not limited to, methyl, ethyl, n-propyl, isopropyl, n-butyl, isobutyl, tert-butyl, n-pentyl, n-hexyl, cyclohexyl, n-heptyl, n-octyl, and ethylhexyl.

The term "aryl" refers to a monovalent group that is aromatic and carbocyclic. The aryl can have one to five rings that are connected to or fused to the aromatic ring. The other ring structures can be aromatic, non-aromatic, or combinations thereof. Examples of aryl groups include, but are not limited to, phenyl, biphenyl, terphenyl, anthryl, naphthyl, acenaphthyl, anthraquinonyl, phenanthryl, anthracenyl, pyrenyl, perylenyl, and fluorenyl.

The terms "free radically polymerizable" and "ethylenically unsaturated" are used interchangeably and refer to a reactive group which contains a carbon-carbon double bond which is able to be polymerized via a free radical polymerization mechanism.

As used herein the term "PVC" is used as a shorthand definition of polyvinyl chloride. The term "phthalate-free PVC" as used herein refers to a PVC material that does not contain phthalate plasticizer.

As used herein the term "plasticizer" refers to a compound(s) which when added to a polymer results in increased flexibility.

As used herein the term "parylene" is a trade name for a variety of chemical vapor deposited poly(p-xylylene) polymers. The term is used herein as a generic name for members of a unique polymer series. The basic member of the series, called Parylene N, is poly-para-xylylene, a completely linear, highly crystalline material.

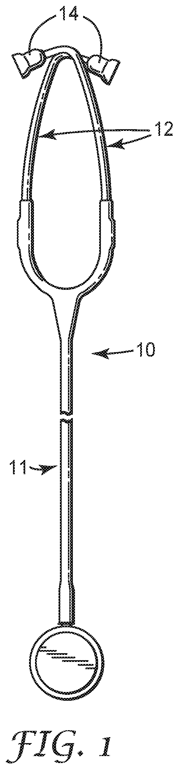

Parylene C, the second commercially available member of the series, is produced from the same monomer modified by the substitution of a chlorine atom for one of the aromatic hydrogens. The structures are shown in *Figure 1**. Parylene N,C & D Chemical Structures.*

Parylene D, the third member of the series, is produced from the same monomer modified by the substitution of the chlorine atom for two of the aromatic hydrogens. Parylene D is similar in properties to Parylene C with the added ability to withstand higher use temperatures.

The parylene also may have one or more hydrogen atoms replaced by fluorine. For example, Parylene AF-4 and parylene VT-4 (generic name, per-fluorinated aromatic ring) also known as PARYLENE HT from SCS.

Parylene X is a crosslinkable parylene. Poly(methyl-p-xylylene) or parylene M
- 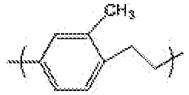 and Parylene E
- 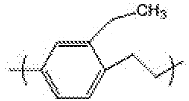 are solvent soluble polymers.

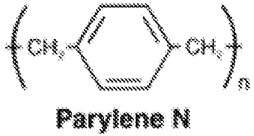

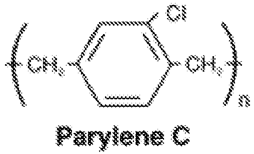

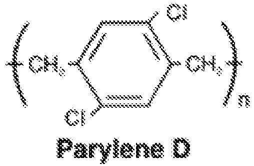

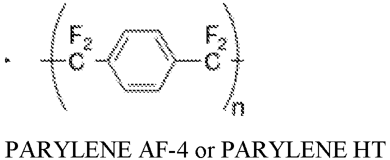

As used herein "parylene copolymers" are chemical vapor deposited derived from p-xylylene and at least one additional ethylenically unsaturated monomer.

As used herein "parylene coatings" refer to coatings of parylene materials.

Disclosed herein are tubes useful to prepare tubing articles, especially medical articles. The tubes have an interior surface and an exterior surface and are prepared from a polymer composition containing one or more extractable components. The tubes contain printing on at least one surface. This printing can contain decoration, information, or a combination thereof. The tubes further have a vapor-deposited coating of a barrier polymer derived from at least one ethylenically unsaturated monomer covering at least the exterior surface of the tube and the printing contained on the surface of the tube. The vapor-deposited barrier polymer coating reduces the extraction of extractable component(s) from the tube and protects the printing. Additionally, the vapor-deposited barrier polymer coating is optically transparent, permitting the printing to be viewed. According to the claimed invention, the barrier polymer comprises a poly(p-xylylene) polymer.

A wide range of polymer compositions containing one or more extractable components are suitable for preparing tubes. In some embodiments, the polymer compositions comprise plasticized polyvinyl chloride (PVC), plasticized PVC copolymers, plasticized polyurethane, or polysiloxanes. Polysiloxanes may or may not include plasticizers like the other classes of polymers, but even if plasticizers are not used in the polysiloxanes, they may contain a variety of extractable materials such as unreacted cyclic siloxane, catalyst residue, tackifier, or a combination thereof. Additionally, any of the above polymer compositions may contain additional materials that may be extractable including stabilizing agents such as thermal stabilizers and UV stabilizers, fillers, and the like.

Polyurethane polymers are well known in the polymer arts, being prepared from the reaction of poly-isocyanates and polyols. A wide range of polyurethane polymers are suitable, including aromatic, aliphatic, urethane block copolymers and copolymers and mixtures thereof. A wide range of plasticizers can be used to plasticize the polyurethane polymers. The polyurethanes may be linear or crosslinked. The polyols used to prepare the polyurethanes may be poly-ether polyols, polyester polyols, aliphatic polyols, siloxane polyols, and the like. The crosslinked polyurethanes are generally made via a 2-part reaction.

Polysiloxane polymers are also well known in the art. Typically, siloxane-based polymers can be prepared in a variety of ways including moisture-curing, hydrosilylation, and condensation reactions. Additionally, a wide range of polysiloxane polymers and polysiloxane precursors are commercially available. As mentioned above, the polysiloxanes can contain a variety of extractable components. Additionally, it can be desirable to apply a barrier coating to polysiloxane tubes, not only as a barrier against leaching of extractable components but also to alter the surface properties of the polysiloxane tube. Polysiloxanes often have a tacky or sticky feel that can be undesirable. Additionally, while efforts are typically expended to ensure that no residual siloxane monomer is present in the polymer that is formed into a tube, these tasks can be labor intensive or expensive and can be avoided through the use of a barrier layer that inhibits or prevents the leaching of extractable material from the polymeric tube.

In many embodiments, the polymer compositions comprise plasticized PVC polymers and plasticized PVC copolymers. PVC copolymers are prepared by copolymerizing one or more ethylenically unsaturated co-monomers with vinyl chloride. An example of a PVC copolymer is polyvinyl chloride-vinyl acetate. Like the PVC polymers described below, the PVC copolymers typically contain plasticizers, often relatively high loadings of plasticizer.

One particularly suitable class of polymers for tubing articles, especially medical tubing articles are PVC polymers. PVC polymers tend to be rigid materials, and therefore plasticizers are added to make the polymers flexible. Typically, the PVC polymer compositions contain a large quantity of plasticizer. The PVC polymer compositions typically contain up to 50-60% by weight plasticizer based on the total weight of the tube composition. In some embodiments, the PVC polymer composition comprises at least 20% by weight plasticizer, typically at least 30% by weight plasticizer, more typically at least 35% by weight plasticizer, or even at least 40% or 45% by weight plasticizer by weight. Generally, the PVC polymer compositions comprise less than 60% by weight plasticizer or less than 55% by weight plasticizer.

As was mentioned above, the use of phthalate-free plasticizers is becoming more prevalent in the preparation of medical articles such as tubing articles. In some embodiments, the polymer composition of the tubes of this disclosure comprise PVC polymer compositions that are free of phthalate plasticizers.

Among the suitable classes of plasticizers are oligomeric or polymeric plasticizers having an average molecular weight of greater than 1000 Daltons, greater than 1500 Daltons, or even greater than 2000 Daltons as determined by GPC with the appropriate standards. Particularly suitable oligomeric or polymeric plasticizers are aliphatic or aromatic polyesters and are available from Hallstar under the PLASTHALL tradename or from Lanxess under the ULTRAMOL brandname.

Another suitable class of plasticizers are sulfonate esters such as MESAMOLL which is an alkylsulphonic acid ester with phenol.

Additional plasticizers include trimellitates such as trimethyl trimellitate, tri-(2-ethylhexyl) trimellitate, tri-(heptyl,nonyl) trimellitate and the like.

Aliphatic dicarboxylic acid-based plasticizers such as bis(2-ethylhexyl)adipate dimethyl adipate, dioctyl adipate, dibutyl sebacate, dibutyl maleate and the like.

Other plasticizers include azelates, benzoates, terephthalates such as dioctyl terephthalate/DEHT (Eastman Chemical Company Trademark: EASTMAN 168), 1,2-Cyclohexane dicarboxylic acid diisononyl ester (BASF trademark: HEXAMOLL DINCH).

Sulfonamides such as N-ethyl toluene sulfonamide (o/p ETSA), ortho and para isomers, glycols and polyethers such as triethylene glycol dihexanoate and tetraethylene glycol diheptanoate.

The polymeric tubes of this disclosure may have a wide range of diameters and thicknesses. Typically, the tubes are not wide tubes, often having an inner diameter of from 1 to 10 millimeters. In some embodiments, the tube has a thickness of from 0.1-7 millimeters. Generally, the tubes are at least 20 centimeters in length, more typically at least 40 centimeters in length.

At least a portion of the surface of the tube article is a printed surface. The printed surface may be a continuous layer so that the printing covers the entire surface, or the printing may be in the form of a pattern. Typically, the printed surface comprises printed pattern. A wide range of printed patterns can be used including printed designs, indicia, geometric patterns, and the like.

A wide range of printing techniques can be used to form the printed surface. In some embodiments, the printing can be carried out using conventional printing techniques such as screen printing and inkjet printing as well a variety of contact printing techniques such as flexographic printing, patterned roll coating, letterpress printing, lithography, stencil printing, and the like. Because the tubes are three-dimensional articles, printing using the above conventional printing techniques can be difficult. One particularly suitable technique is hydrographic printing.

Hydrographic printing, also known as water transfer printing and immersion printing, is a method of applying printed designs to three-dimensional surfaces. In the process, a polyvinyl alcohol hydrographic film, which has been gravure-printed with the graphic image to be transferred, is carefully placed on the water's surface in a dipping tank. The clear film is water-soluble, and dissolves after applying an activator solution. Once dipping is begun, the surface tension of the water will allow the pattern to curve around any shape. Any remaining residue is then rinsed off thoroughly. The ink adheres to the desired surface and it cannot be washed off easily. It is then allowed to dry.

To the printed surface of the article, at least one ethylenically unsaturated monomer is vapor-deposited to form a polymer layer. This vapor-deposited polymer layer is an optically transparent barrier layer. According to the claimed invention, the polymer barrier layer comprises parylene or a copolymer of parylene. Parylene is the trade name for vapor-deposited poly-para-xylylene. Poly para-xylylene, also referred to as parylene N, is shown in Formula 1 below:

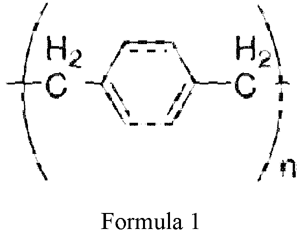

Parylene is prepared when the precursor [2,2]paracyclophane (shown in Formula 2 below) is heated above 550°C in vacuum. Upon condensation on a surface, the poly-para-xylylene forms.

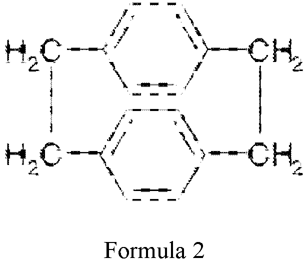

In some embodiments, the vapor-deposited coating of a crosslinked vinyl polymer comprises parylene, that is to say parylene N. In other embodiments, the vapor-deposited coating of a crosslinked vinyl polymer comprises a substrituted parylene such as parylene C. Parylene C has the general structure shown in Formula 3 below. Parylene C is prepared by using a chlorine-substituted dimer.

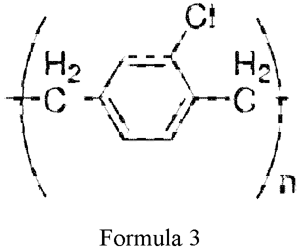

In some embodiments, the vapor-deposited coating of a barrier polymer comprises a copolymer of parylene and at least one (meth)acrylate. A wide variety of (meth)acrylates are suitable. In some embodiments, the (meth)acrylate comprises at least one C₃-C₁₈ alkyl (meth)acrylate.

The vapor-deposited coating of a barrier polymer can have a wide range of thicknesses. As mentioned above, the coating may be a continuous coating, present on the entire exterior surface of the tubing or it can be present in selective regions of the exterior surface of the tubing. Additionally, the coating may be present on both the interior and exterior surfaces of the tubing. In some embodiments, the vapor-deposited barrier polymer coating has a thickness of from 0.2-10 micrometers. In other embodiments, the vapor-deposited crosslinked vinyl polymer coating has a thickness of from 1-5 micrometers. The coating may have a uniform or essentially uniform thickness, or the coating thickness may vary over the surface of the tubing. Typically, the coating has an essentially uniform thickness.

The vapor-deposited coating of a barrier polymer may comprise additional additives. Among the additives are heat stabilizers, coloring agents, mold release agents, and antimicrobial agents.

One necessary feature of the barrier polymers is their ability to strongly adhere to the tube. This is complicated by the fact that typically the tubing polymeric compositions contain high levels of plasticizer. Among the circumstances that make strong adhesion necessary is in bending. It is desirable that the barrier polymers are able to withstand 180° bending of the tubing for at least 5,000 cycles, more desirably at least 10,000 cycles and even more desirably at least 20,000 cycles when tested according to a "Bend Test" as is understood in the art.

The vapor-deposited coatings of a barrier polymer of the current disclosure reduce the extractability of the extractable components of the polymer compositions of the tubing and also protect the printing. In some embodiments, the coatings prevent the extraction of extractable components from the polymer compositions of the tubing. This extraction refers to a variety of extraction methods. It is particularly desirable that the barrier polymers prevent extraction by both polar fluids such as aqueous and hydroalcoholic (e.g. rubbing alcohol, 70/30 v/v isopropanol water) disinfectants as well as nonpolar fluids such as skin oil (sebum) and synthetic sebum. Examples of extraction methods include solvent extraction, heat extraction, and skin contact extraction. Solvent extraction involves the application of solvent to the polymer composition surface. Suitable solvents are described above and include polar fluids and nonpolar fluids. Upon removal of the solvent by wiping or similar techniques results in extractable components leaving the polymer composition. Heat extraction involves applying heat to the polymer composition which results in the extractable components leaching from the polymer composition. Heat can be used alone or in combination with the solvent extraction method described above. Skin contact extraction results from the contact of skin to the polymer composition where the extractable components in the polymer composition leach from the polymer composition. In practice, medical devices containing tubing articles can encounter each of these extraction methods, or a combination of them. The tubing article may be washed or cleaned with a solvent or other liquid, or the tubing may come into contact with a variety of fluids. Heat extraction can occur from contact with, for example, body heat, or higher temperatures such as, for example 50°C if the article is left in a vehicle on a hot day. Similarly, skin contact extraction can occur from exposure of the tubing article to skin.

The extractability of extractable components from a polymer composition can be modeled in a variety of ways as is well understood in the art.

Besides the reduction of extractability of the extractable components of the polymer composition of the tubing, the coatings also protect the printing on the surface on the tubing article. In some embodiments, the vapor-deposited coating changes the texture, the coefficient of friction, or a combination thereof. This can be particularly true of polysiloxane tubes which, as described above, can have a tacky or sticky feel.

The coated tubing of the current disclosure can be used to prepare a wide variety of medical articles. One particularly suitable use is for the tubing of stethoscopes. A typical stethoscope is shown in Figure 1. Figure 1 shows a top view of stethoscope **10.** Stethoscope **10** has tubing **11** attached to dual sound transmitting tubes **12,** terminating in ear tips **14.** The elements of stethoscope **10** can vary from those shown, but the fundamental elements are present. This disclosure relates to tubing **11.** The reduction of extractability of plasticizer desirable features of the tubing **11.** Among these features are flexibility, handleability, and pleasing aesthetics. In many cases tubing **11** is prepared from plasticizer-filled PVC polymer. Flexibility is imparted to the PVC polymer by high levels of plasticizer, and retention of this plasticizer within the polymer matrix, in other words the lack of plasticizer extraction, is important to the usefulness of the tubing. Because the PVC polymer, or other polymeric material if used, often have high levels of plasticizer or other extractable components, migration of the plasticizer out of the polymer can occur over time, or the plasticizer can be extracted by washing or cleaning, or by contact with skin either when worn or when handled. Loss of flexibility of the tubing reduces the usefulness of the stethoscope. Handleability relates to the feel of the tubing, which is going to be worn and handled by the user of the stethoscope. Another important feature of handleability also relates to the lack of plasticizer extraction in that if plasticizer exudes from the tubing, the feel of the tubing becomes unpleasant and plasticizer can be transferred to the user's hand. A variety of aesthetic features are desirable including the printed patterns, color, texture, gloss, and feel are desirable for the tubing of the stethoscope. Because the tubing comes into frequent contact with skin, the texture and feel of the tubing should be aesthetically pleasant, meaning that the tubing should be smooth and have a low coefficient of friction.

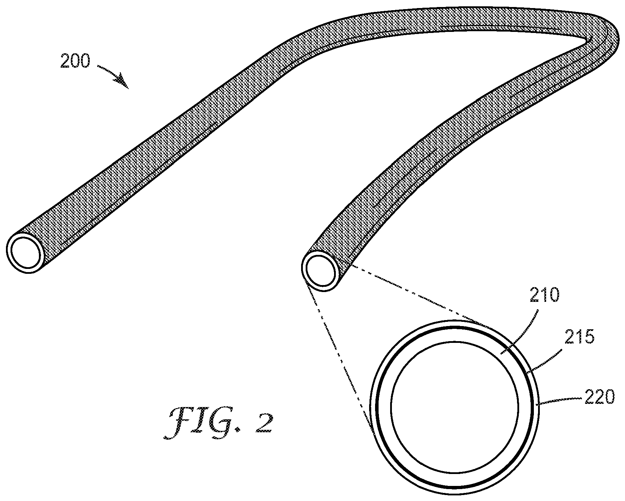

Figure 2 shows a cross-sectional view of a tubing article of this disclosure. Tubing article **200** has tubing layer **210** with printed layer **215** and transparent coating layer **220.** Printed layer **215** is shown printed as one pattern, but it can be printed in a wide variety of patterns and designs. Transparent coating layer **220** is a vapor-coated barrier layer.

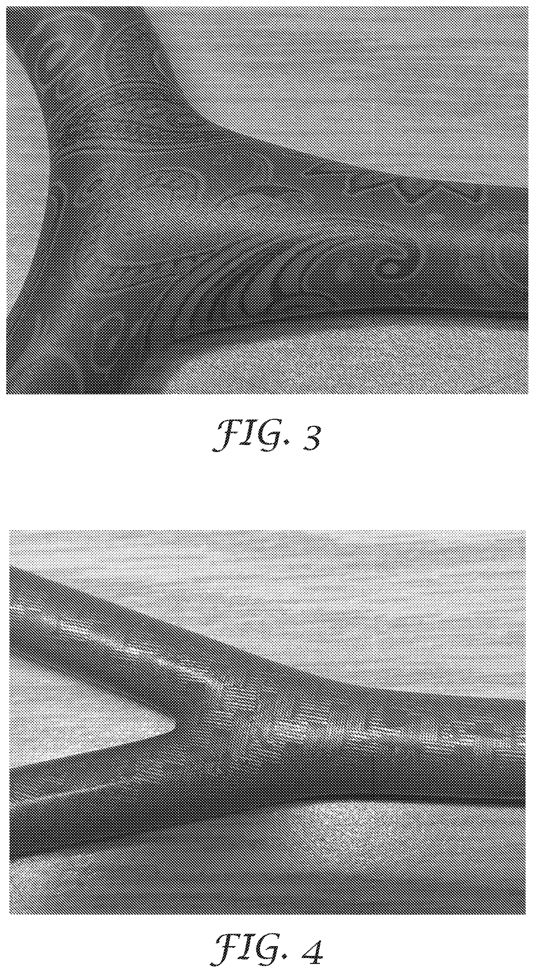

Figures 3 and 4 are top views of pictures of tubing that has been printed and vapor-coated.

As mentioned above, the coated tubing of the present disclosure provides these desirable features. The coatings not only inhibit the extraction of plasticizer or other extractable components from the polymeric tubing and protect the printing, but also the coatings can provide desirable handleability and aesthetic features.

Also disclosed are methods for preparing medical articles. The method comprises providing a tube with an interior surface and an exterior surface, printing on at least a portion of the exterior surface of the tube, and vapor coating a barrier polymer derived from at least one ethylenically unsaturated monomer onto at least a portion of the exterior surface of the tube, the barrier polymer defining a barrier polymer coating that is optically transparent, and wherein the barrier polymer comprises a poly(p-xylylene) polymer. The barrier polymer may be coated on the entire exterior surface of the tube and may also be coated on the interior surface of the tube, as has been described above. The polymeric tubing has been described above and comprises a polymer composition containing one or more extractable components.

In some embodiments, printing on at least a portion of the exterior surface of the tube comprises hydrographic printing. In some embodiments, the printing is a continuous layer. More typically, the printing is the form of a pattern, as described above.

In some embodiments, vapor coating a barrier polymer onto at least a portion of the exterior surface of the tube comprises placing the tube in a deposition chamber, applying a vacuum to the deposition chamber, heating a precursor material to volatize and optionally pyrrolyze at least a portion of it, and introducing the volatilized and optionally pyrrolyzed percursor material into the deposition chamber. The precursor material comprises the at least one ethylenically unsaturated monomer. According to the claimed invention, the precursor material comprises parylene or a mixture of parylene and at least one (meth)acrylate. Suitable parylene compositions are described above. As was described above, the volatilized precursor material deposits on the tubing and polymerizes to form the barrier coating on the tubing surface. At least a portion of the exterior surface of the tubing is coated, and in many embodiments, the entire exterior surface of the tubing is coated. In some embodiments, both the interior and exterior surface of the tubing is coated. The vapor-deposited coating is optically transparent.

There are a variety of methods for placing the tubing in the deposition chamber. In some embodiments, the tubing may be placed on a rotating basket or it may be hung from a rack in the deposition chamber.

The tubing suitable for use in the methods of this disclosure are polymeric compositions with extractable components. Examples of suitable polymeric compositions have been described above. In some embodiments, the polymer compositions comprise plasticized polyvinyl chloride (PVC), plasticized PVC copolymers, plasticized polyurethane, or polysiloxanes. Polysiloxanes may or may not include plasticizers like the other classes of polymers, but even if plasticizers are not used in the polysiloxanes, they may contain a variety of extractable materials such as unreacted cyclic siloxane, catalyst residue, tackifier, plasticizer, or a combination thereof. Additionally, any of the above polymer compositions may contain additional materials that may be extractable including stabilizing agents such as thermal stabilizers and UV stabilizers, fillers, and the like.

The polymeric tubes of this disclosure may have a wide range of diameters and thicknesses. Typically, the tubes are not wide tubes, often having an internal diameter of from 1 to 10 millimeters. In some embodiments, the tube has a thickness of from 0.1-7 millimeters. The tube may have a variety of lengths, typically at least 20 centimeters, often at least 40 centimeters.

The vapor-deposited crosslinked polymer coatings have been described above. Typically, the polymer coating has a thickness of from 0.2-10 micrometers. In some embodiments, the polymer coating has a thickness of from 1-5 micrometers.

As mentioned above, the coated tubing can be used to prepare a wide variety of medical articles. In some embodiments, the coated tubes are used in stethoscopes, as has been described above.

The tubes of this disclosure also have a vapor-deposited coating of a barrier polymer derived from at least one ethylenically unsaturated monomer covering at least a portion of the exterior surface of the tube. According to the claimed invention this barrier polymer comprises a poly(p-xylylene) polymer. For example, stethoscope tubing is desirably coated at least along the part of the tubing which would contact the skin when worn around the neck. In many embodiments, the entire exterior surface of the tube has a vapor-deposited coating of a barrier polymer. In some embodiments, the tube also has a vapor-deposited coating of a barrier polymer on the interior surface of the tube.

### Examples

These examples are merely for illustrative purposes only and are not meant to be limiting on the scope of the appended claims. All parts, percentages, ratios, etc. in the examples and the rest of the specification are by weight, unless noted otherwise.

### Examples 1 and 2

Stethoscope tubes which were hydrographically printed and coated with a parylene coating are shown in Figure 3 (Example 1) and Figure 4 (Example 2). The preparation of these samples is presented below.

### Sample Preparation

### Printing Process

The stethoscope tubes used in these Examples were polyvinyl chloride (PVC) sound transmission tubes prepared with a phthalate-free poly(vinyl chloride) (PVC) formulation, and are used in the manufacture of LITTMANN Cardiology stethoscopes (3M Company, St. Paul, MN). The PVC plastisol formulations were made with different color pigments that provided dip coated PVC sound transmission tubes with similar properties. Both color tubes were flexible and elastomeric.

The tubes were hydrographically printed with a paisley pattern (Example 1) and a silver weave pattern (Example 2) using Liquid Print, Inc. materials. In the process, a polyvinyl alcohol hydrographic film was gravure-printed with the graphic image to be transferred, and was carefully placed on the water surface in a dipping tank. The polyvinyl alcohol film was water-soluble, and dissolves after applying an activator solution. Upon dipping the tubes into the dipping tank, the surface tension of the water allowed the pattern to curve around the tube surface. The residue was rinsed off and the tube was allowed to dry.

### Parylene Coating Process

The dried, printed sound transmission tubes were allowed to dry prior loaded into a parylene-coating reactor. The tubes were coated using a vacuum process at approximately 160 milliTorr while either statically hung from a rotating carousel or while tumbled in a cylindrical wire cage. Sample lots of the tubes were coated with 0.8 micrometer thick coatings of Parylene-C. The coating process coated both interior and exterior surfaces of the tubing.

Prior to printing and parylene coating, the PVC sound transmission tubes had a glossy finish and slightly tacky feel with a relatively high coefficient of friction. After application of the Parylene-C coating, the Parylene-coated sound transmission tubes had a dry, slippery feel and a lower coefficient of friction.

## Claims

1. A medical article (200) comprising:
a tube (210) with an interior surface and an exterior surface, comprising a polymer composition containing one or more extractable components, and wherein at least a portion of the exterior surface of the tube is a printed surface; and
a transparent vapor-deposited coating (220) of a barrier polymer derived from at least one ethylenically unsaturated monomer and covering at least a portion of at least the exterior surface of the tube, such that the printed surface is visible through the barrier polymer,
wherein the barrier polymer comprises a poly(p-xylylene) polymer, and the barrier polymer is optionally crosslinked.

2. The medical article of claim 1, wherein the polymer composition containing one or more extractable components comprises:
plasticized polyvinyl chloride (PVC);
plasticized PVC copolymers;
plasticized polyurethane; or
polysiloxane containing unreacted cyclic siloxane, catalyst residue, plasticizer, tackifier, or a combination thereof.

3. The medical article of claim 1, wherein the printed surface is formed by hydrographic printing.

4. The medical article of claim 1, wherein the printed surface comprises a continuous layer or a pattern, wherein the pattern comprises printed designs, indicia, geometric patterns or combinations thereof.

5. The medical article of claim 1, wherein poly(p-xylylene) polymer comprises parylene N, parylene C, parylene D, or parylene HT.

6. The medical article of claim 1, wherein the transparent vapor-deposited coating has a thickness of from 0.2-10 micrometers.

7. The medical article of claim 1, wherein the polymer composition containing one or more extractable components has a thickness of from 0.1-7 millimeters.

8. The medical article of claim 1, wherein the tube comprises tubing for a stethoscope.

9. The medical article of claim 1, wherein the extractable components are extractable by solvent extraction using a polar or nonpolar solvent.

10. The medical article of claim 1, wherein the barrier polymer comprises a copolymer of a poly(p-xylylene) and at least one (meth)acrylate.

11. The medical article of claim 10, wherein the at least one (meth)acrylate comprises at least one C₃-C₁₈ alkyl (meth)acrylate.

12. A method of preparing a medical article comprising:
providing a tube with an interior surface and an exterior surface, comprising a polymer composition containing one or more extractable components;
printing a printed surface on at least a portion of the exterior surface of the tube; and
vapor coating a barrier polymer derived from at least one ethylenically unsaturated monomer onto at least a portion of at least the exterior surface of the tube, the barrier polymer defining a barrier polymer coating that is optically transparent;
wherein the barrier polymer comprises a poly(p-xylylene) polymer, and the barrier polymer is optionally crosslinked, and optionally, wherein the tube comprises tubing for a stethoscope.

13. The method of claim 12, wherein printing comprises hydrographic printing.

14. The method of claim 12, wherein vapor coating the barrier polymer onto at least a portion of at least the exterior surface of the tube comprises:
placing the tube in a deposition chamber;
applying a vacuum to the deposition chamber;
heating a precursor material to volatize at least a portion of the precursor material and form a volatilized precursor material; and
introducing the volatilized precursor material into the deposition chamber, wherein the precursor material comprises a p-xylylene or a mixture of a p-xylylene and at least one (meth)acrylate.

15. The method of claim 12, wherein the barrier polymer coating has a thickness of from 0.2-10 micrometers.

## Patentansprüche

1. Ein medizinischer Artikel (200), aufweisend:
einen Schlauch (210) mit einer Innenoberfläche und einer Außenoberfläche, aufweisend eine Polymerzusammensetzung, die eine oder mehrere extrahierbare Komponenten enthält, und wobei mindestens ein Teil der Außenoberfläche des Schlauchs eine gedruckte Oberfläche ist; und
eine transparente aufgedampfte Beschichtung (220) aus einem Barrierepolymer, das von mindestens einem ethylenisch ungesättigten Monomer abgeleitet ist und mindestens einen Teil von mindestens der Außenoberfläche des Schlauchs bedeckt, sodass die gedruckte Oberfläche durch das Barrierepolymer hindurch sichtbar ist,
wobei das Barrierepolymer ein Poly-(p-xylylen)polymer aufweist und das Barrierepolymer optional vernetzt ist.

2. Der medizinische Artikel nach Anspruch 1, wobei die Polymerzusammensetzung, die eine oder mehrere extrahierbare Komponenten enthält, aufweist:
weichgemachtes Polyvinylchlorid (PVC);
weichgemachte PVC-Copolymere;
weichgemachtes Polyurethan; oder
Polysiloxan, das nicht umgesetztes zyklisches Siloxan, Katalysatorrückstände, Weichmacher, Klebrigmacher oder eine Kombination davon enthält.

3. Der medizinische Artikel nach Anspruch 1, wobei die gedruckte Oberfläche durch hydrografisches Drucken gebildet ist.

4. Der medizinische Artikel nach Anspruch 1, wobei die gedruckte Oberfläche eine kontinuierliche Schicht oder ein Muster aufweist, wobei das Muster gedruckte Designs, Zeichen, geometrische Muster oder Kombinationen davon aufweist.

5. Der medizinische Artikel nach Anspruch 1, wobei das Poly-(p-xylylen)polymer Parylen N, Parylen C, Parylen D oder Parylen HT aufweist.

6. Der medizinische Artikel nach Anspruch 1, wobei die transparente aufgedampfte Beschichtung eine Dicke von 0,2-10 Mikrometer hat.

7. Der medizinische Artikel nach Anspruch 1, wobei die Polymerzusammensetzung, die eine oder mehrere extrahierbare Komponenten enthält, eine Dicke von 0,1-7 Millimeter hat.

8. Der medizinische Artikel nach Anspruch 1, wobei der Schlauch Schlauchmaterial für ein Stethoskop aufweist.

9. Der medizinische Artikel nach Anspruch 1, wobei die extrahierbaren Komponenten durch Lösungsmittelextraktion unter Verwendung eines polaren oder unpolaren Lösungsmittels extrahierbar sind.

10. Der medizinische Artikel nach Anspruch 1, wobei das Barrierepolymer ein Copolymer aus einem Poly-(p-xylylen) und mindestens einem (Meth)acrylat aufweist.

11. Der medizinische Artikel nach Anspruch 10, wobei das mindestens eine (Meth)acrylat mindestens ein C3-C18-Alkyl(meth)acrylat aufweist.

12. Ein Verfahren zum Herstellen eines medizinischen Artikels, aufweisend:
Bereitstellen eines Schlauchs mit einer Innenoberfläche und einer Außenoberfläche, aufweisend eine Polymerzusammensetzung, die eine oder mehrere extrahierbare Komponenten enthält;
Drucken einer gedruckten Oberfläche auf mindestens einen Teil der Außenoberfläche des Schlauchs; und
Aufdampfen eines Barrierepolymers, das von mindestens einem ethylenisch ungesättigten Monomer abgeleitet ist, auf mindestens einen Teil von mindestens der Außenoberfläche des Schlauchs, wobei das Barrierepolymer eine Barrierepolymerbeschichtung definiert, die optisch transparent ist;
wobei das Barrierepolymer ein Poly-(p-xylylen)polymer aufweist und das Barrierepolymer optional vernetzt ist und wobei der Schlauch optional Schlauchmaterial für ein Stethoskop aufweist.

13. Das Verfahren nach Anspruch 12, wobei das Drucken hydrografisches Drucken aufweist.

14. Das Verfahren nach Anspruch 12, wobei das Aufdampfen des Barrierepolymers auf mindestens einen Teil von mindestens der Außenoberfläche des Schlauchs aufweist:
Platzieren des Schlauchs in einer Abscheidungskammer;
Anlegen eines Vakuums an die Abscheidungskammer;
Erwärmen eines Vorläufermaterials, um mindestens einen Teil des Vorläufermaterials zu verflüchtigen und ein verflüchtigtes Vorläufermaterial zu bilden; und
Einführen des verflüchtigten Vorläufermaterials in die Abscheidungskammer, wobei das Vorläufermaterial ein p-Xylylen oder eine Mischung aus einem p-Xylylen und mindestens einem (Meth)acrylat aufweist.

15. Das Verfahren nach Anspruch 12, wobei die Barrierepolymerbeschichtung eine Dicke von 0,2-10 Mikrometer hat.

## Revendications

1. Article médical (200) comprenant :
un tube (210) avec une surface intérieure et une surface extérieure, comprenant une composition de polymère contenant un ou plusieurs composants extractibles, et dans lequel au moins une partie de la surface extérieure du tube est une surface imprimée ; et
un revêtement transparent déposé en phase vapeur (220) d'un polymère barrière dérivé d'au moins un monomère à insaturation éthylénique et recouvrant au moins une partie d'au moins la surface extérieure du tube, de telle sorte que la surface imprimée est visible à travers le polymère barrière,
dans lequel le polymère barrière comprend un polymère de poly(p-xylylène), et le polymère barrière est éventuellement réticulé.

2. Article médical selon la revendication 1, dans lequel la composition de polymère contenant un ou plusieurs composants extractibles comprend :
du poly(chlorure de vinyle) (PVC) plastifié ;
des copolymères de PVC plastifiés ;
du polyuréthane plastifié ; ou
du polysiloxane contenant du siloxane cyclique n'ayant pas réagi, un résidu de catalyseur, un plastifiant, un agent collant, ou une combinaison de ceux-ci.

3. Article médical selon la revendication 1, dans lequel la surface imprimée est formée par impression hydrographique.

4. Article médical selon la revendication 1, dans lequel la surface imprimée comprend une couche continue ou un motif, dans lequel le motif comprend dessins imprimés, repères, motifs géométriques ou combinaisons de ceux-ci.

5. Article médical selon la revendication 1, dans lequel le polymère de poly(p-xylylène) comprend du parylène N, du parylène C, du parylène D, ou du parylène HT.

6. Article médical selon la revendication 1, dans lequel le revêtement transparent déposé en phase vapeur a une épaisseur allant de 0,2 à 10 micromètres.

7. Article médical selon la revendication 1, dans lequel la composition de polymère contenant un ou plusieurs composants extractibles a une épaisseur allant de 0,1 à 7 millimètres.

8. Article médical selon la revendication 1, dans lequel le tube comprend une tubulure pour un stéthoscope.

9. Article médical selon la revendication 1, dans lequel les composants extractibles peuvent être extraits par extraction par solvant à l'aide d'un solvant polaire ou non polaire.

10. Article médical selon la revendication 1, dans lequel le polymère barrière comprend un copolymère d'un poly(p-xylylène) et d'au moins un (méth)acrylate.

11. Article médical selon la revendication 10, dans lequel l'au moins un (méth)acrylate comprend au moins un (méth)acrylate d'alkyle en C3-C18.

12. Procédé de préparation d'un article médical comprenant :
la fourniture d'un tube avec une surface intérieure et une surface extérieure, comprenant une composition de polymère contenant un ou plusieurs composants extractibles ;
l'impression d'une surface imprimée sur au moins une partie de la surface extérieure du tube ; et
le revêtement en phase vapeur d'un polymère barrière dérivé d'au moins un monomère à insaturation éthylénique sur au moins une partie d'au moins la surface extérieure du tube, le polymère barrière définissant un revêtement de polymère barrière qui est optiquement transparent ;
dans lequel le polymère barrière comprend un polymère de poly(p-xylylène), et le polymère barrière est éventuellement réticulé, et éventuellement, dans lequel le tube comprend une tubulure pour un stéthoscope.

13. Procédé selon la revendication 12, dans lequel l'impression comprend une impression hydrographique.

14. Procédé selon la revendication 12, dans lequel le revêtement en phase vapeur du polymère barrière sur au moins une partie d'au moins la surface extérieure du tube comprend :
le placement du tube dans une chambre de dépôt ;
l'application d'un vide à la chambre de dépôt ;
le chauffage d'un matériau précurseur pour volatiliser au moins une partie du matériau précurseur et former un matériau précurseur volatilisé ; et
l'introduction du matériau précurseur volatilisé dans la chambre de dépôt, dans lequel le matériau précurseur comprend un p-xylylène ou un mélange d'un p-xylylène et d'au moins un (méth)acrylate.

15. Procédé selon la revendication 12, dans lequel le revêtement de polymère barrière a une épaisseur allant de 0,2 à 10 micromètres.
